# EUROPEAN PATENT APPLICATION

(11) **EP 4 180 065 A1**
(43) Date of publication of application: **17.05.2023**
(21) Application number: 21832016.6
(22) Date of filing: 30.06.2021
(51) Int. Cl.: A61L 9/20, A61L 2/10, C02F 1/32

(54) **UVC IRRADIATION TREATMENT CONTAINER**

(30) Priority: 01.07.2020 JP 2020125801
(71) Applicant: Oyama, Nobuo, Yokohama shi, Kanagawa 227-0067 (JP)
(72) Inventor: Oyama, Nobuo, Yokohama shi, Kanagawa 227-0067 (JP)
(74) Representative: Manitz Finsterwald Patent- und Rechtsanwaltspartnerschaft mbB
(86) International application number: PCT/JP2021/025705
(87) International publication number: WO 2022/004899

(57) **Abstract**

To implement UVC energy irradiation at a unified amount to a target for sterilization and decomposition of all in a fluid that is flowing, by greatly increasing a depth of a fluid that receives radiation from a UVC light source significantly to increase a rate at which UVC energy per unit irradiates the target of sterilization and decomposition in the fluid, in continuous sterilization of fluids by UVC irradiation in a container optically shielded from the outside. In order to allow the entire fluid flowing into the container to flow out of the container with the same in-container movement time, the container is equipped with one or a plurality of bulkheads composed of highly UVC permeable material at an angle to receive a maximum radiation from the UVC light source; an opening at one end through which the fluid passes is disposed to be positioned at an end of an opposite side mutual next to the other thereby allowing the fluid flowing into the container to flow in layers along the bulkheads; bending one after the other from the openings in the bulkheads to further flow layers forms a series of overlapping flow layers over the entire area of the container, irradiating the UVC through these layers.

## Description

### [Technical Field]

This invention relates to a method for continuous sterilization of fluids by irradiating air or water with C-region ultraviolet light (hereinafter, "UVC").

### [Background Art]

A fluid-sterilization device has been commercially available as of April 2020. It uses a single cylindrical UVC lamp positioned at the center of a cylindrical container optically shielded from an external environment from indoor air and water in piped transport; fluid is continuously passed between it and an inner wall of the cylindrical container, with UVC irradiation of the fluid being implemented over the passage time. However, because of their low energy efficiency, these devices are limited to very low treatment capacities.

Conversely, research institutes, universities, and companies in Japan have been conducting R&D to improve the efficiency and longevity of UVC LEDs, and successful examples have been published. The shape and radiation direction of UVC LEDs make them unsuitable for a sterilizer that implements UVC irradiation in the cylindrical containers described above. A sterilizing method that uses a container whose basic inside shape is parallelepiped has been considered.

### [Citation List]

### [Non-patent Document]

[Non-patent document 1] "Development of Deep UV LEDs for Sterilization;" Hideki Hirayama, Senior Researcher, Hirayama Quantum Optical Device Laboratory, Riken, National Research and Development Agency.
[Non-patent document 2] Y's Company, Inc. "UV Irradiation Water Sterilization Equipment (Pipe-Type)" .

### [Summary of Invention]

### [Technical Problem]

With the fluid sterilization equipment, in the method of implementing UVC irradiation while the fluid to be sterilized is continuously passed through a small-diameter cylindrical container described in [0002] above, the volume ratio of bacteria and other microbes and viruses in the fluid (hereinafter, "target of sterilization and decomposition") is extremely small, and because the UVC absorption rate of water and air is extremely low compared to the structural material of the container, most of the UVC energy irradiated to the fluid is repeatedly reflected and absorbed in the cylindrical container (hereinafter, "cylindrical treatment container" or "treatment container") in which the UVC irradiation treatment is implemented, and is absorbed by the structural material of the treatment container.

To reduce the rate at which UVC energy is absorbed by such non-purpose materials, a diameter of the treatment container was increased, and a rate of increase in volume was increased compared to the rate of increase in an area of its inner walls. This means that an amount of fluid present in the treatment container and an amount of sterilization and decomposition target present in the treatment container along with that fluid increase at a similar rate, while a rate at which the irradiated UVC energy is absorbed by the inner walls of the treatment container decreased, thereby increasing an amount of energy irradiating these targets for sterilization and decomposition.

However, as described in [0006], when the inner diameter of the cylindrical treatment container is increased and the volume of fluid in the treatment container is increased, a partial flow with a higher velocity than a majority of the fluid in the treatment container, called a "flow path" between the outlet and inlet, is easily generated because a difference between the inner diameter of the treatment container and the inner diameter of the fluid outlet widens.

In the event that this "flow path" is generated, it is impossible to align the speed of the fluid's passing through the treatment container to the UVC irradiation intensity, so turbulence is generated in the flow of the fluid in the treatment container, and although it is possible to prevent generation of the "flow path," it becomes difficult to cause adequate turbulence throughout the entire treatment container along with the increased difference in the inner diameter of the treatment container and the inner diameter of the outlet.

Conversely, the treatment container of the fluid sterilizer mentioned in [0003] is a UVC irradiation treatment container that is optically shielded from an external environment and whose basic shape inside is a rectangular parallelepiped (hereinafter, "parallelepiped-type treatment container" or "treatment container"). Sterilization of the fluid is implemented by UVC irradiation of the fluid that continuously passes through the treatment container by a light source such as a large number of UVC LED chips, UVC LED lamps, or conventional cylindrical UVC lamps (hereinafter, "UVC light source" or "light source") installed side by side across an entire surface of one of its inner surfaces.

In a parallelepiped treatment container, as a method to reduce the same problems as described in [0005] above and to attain the same effect as described at [0006], a distance between an array surface on the UVC irradiation side of the light source (hereinafter, "light source array surface") and an inner wall facing thereto (hereinafter, "inner wall opposing the light source") described in [0009] above is significantly increased thereby significantly increasing an amount of fluid in the treatment container, and the difficulty described at [0007] is generated even when attaining a similar effect to that described in [0006].

This invention solves the difficulties described in [0007] and [0010] in the parallelepiped treatment container and cylindrical treatment container.

### [Solution to Problem]

The means of solving the problem in the parallelepiped treatment container described in [0009] above is to significantly widen the gap between the light source array surface and the inner wall opposing the light source, to form a layer of flow that substantially expands the width of the entire surface, parallel to the fluid that flows from one end of the treatment container to the light source array surface. The structure is designed to receive UVC irradiation from the light source array surface through the multiple overlapping layers of flow, while the fluid flowing in from one end of the processing container forms a layer of flow parallel to the light source array surface and almost as wide as its entire surface.

The solution to the problem of cylindrical treatment containers is to have a functional structure in which the fluid flowing in from one end of the container forms an annular-shaped flow layer surrounding a cylindrical UVC lamp installed in the center of the container and then bends one after another into similar flow layers, forming a single, interconnected flow layer that overlaps with each other throughout the entire area of the container. The functional structure of the UVC lamp is such that the UVC irradiation from the lamp is received through the multiple overlapping layers of flow.

In order to obtain the fluid flow described in [0012] above in a parallelepiped-type treatment container, a parallelepiped faced bulkhead 7a made of a plate-shaped UVC highly permeable material or a sheet or film-shaped UVC highly permeable material attached to a rigid parallelepiped frame is placed between the light source array surface 6 and the inner wall opposing the light source, and the three end faces of the partition 7a are made to be in close contact with the inner wall of the enclosure 1a or the inner wall of the treatment container, and the remaining end faces are made to have a predetermined spacing between them and the inner wall, forming an opening through which the fluid can pass, and in the case of multiple bulkheads 7a, the enclosure 1a of the treatment container is provided with dimensions having a predetermined interval parallel to each other between the light source array surface 6 and the nearest bulkhead 7a, between the bulkheads 7a each other, and between the inner wall opposite the light source and the nearest bulkhead 7a, and between adjacent bulkheads 7a, the above openings are formed between the ends opposite to each other and the inner wall, so that they are attached to the enclosure 1a of said processing container at the above predetermined intervals.

Furthermore, on the inner wall surface opposite the inner wall on the opening side of the bulkhead 7a immediately adjacent to the light source array surface 6 (hereinafter, "light source mounting surface 5") or on the part sandwiched between the light source array surface 6 and the surface of the bulkhead 7a immediately adjacent thereto, an opening of the same shape and dimension, or of approximately the same area, is provided in the processing container, and furthermore, an opening is provided in the same shape and dimension, or in approximately the same area, as that portion of the processing container.

Conversely, an opening of the same shape and size, or of approximately the same area, communicating to an outside of the treatment container is disposed in a portion sandwiched between the inner wall opposing light source and the nearby bulkhead 7a on an inner wall opposite the opening in the bulkhead 7a closest to the light source opposing inner wall. Conversely, alternatively, a similar opening communicating to an outside of the container is disposed at an end opposite the opening side of the bulkhead 7a on the inner wall opposing light source.

One of the openings communicating to the outside of the treatment container described in [0015] and [0016] above is an outlet 2a for the fluid from the treatment container and the other is an inlet 4a of the fluid into the treatment container, and to this inlet 4a is connected a duct that wraps around the outside of the treatment container, an inlet 3a for the fluid is disposed at an end of this duct, and the fluid intake 3a is connected to the duct.

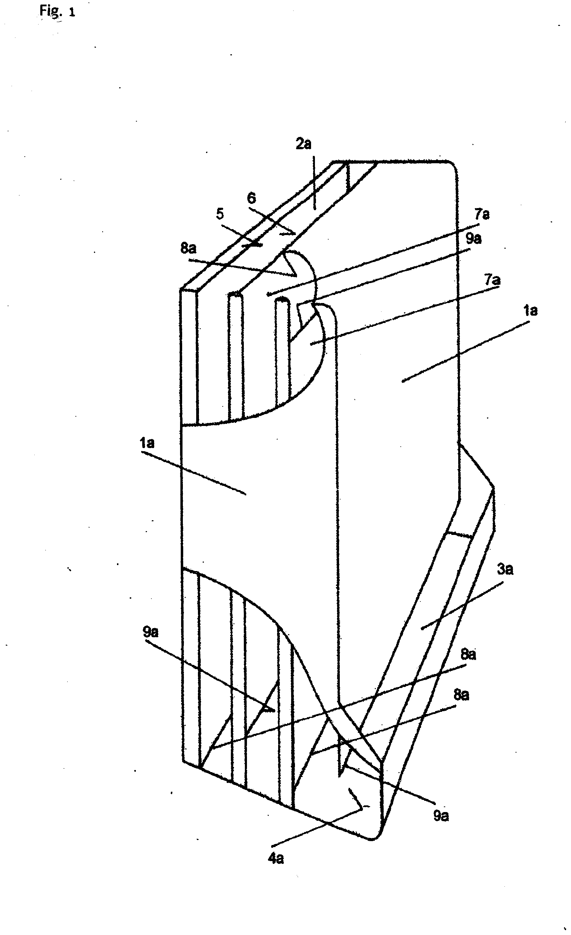

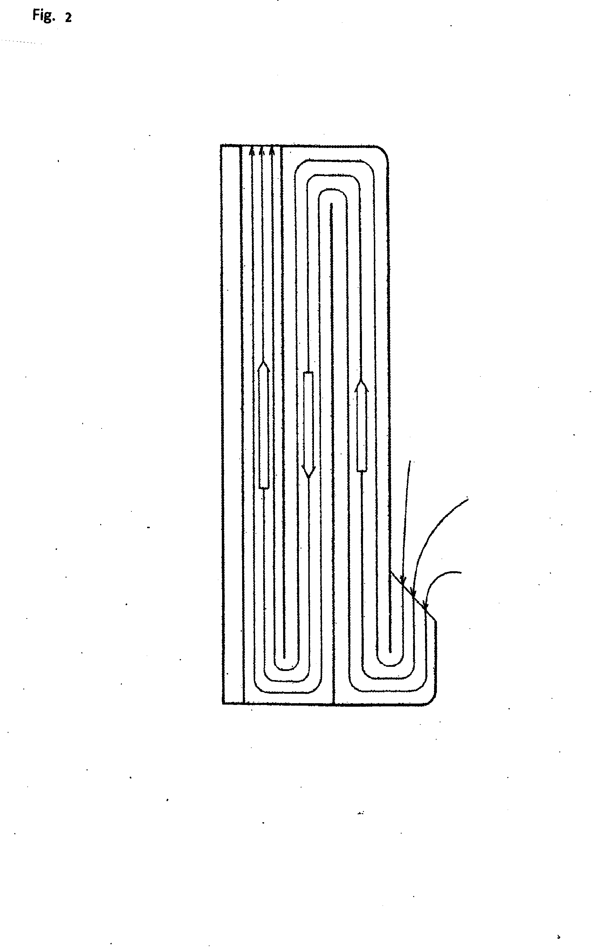

Fig. 1 is a schematic view of a parallelepiped-type treatment container when the fluid targeted for sterilization by UVC irradiation (hereinafter, "target fluid") is air. A light source mounting surface 5 and a light source array surface 6, two bulkheads 7a, the air outlet 2a, the air inlet 4a, and the air intake 3a are disposed at one inner wall of a enclosure 1a. Fig. 2 is a general view of air flowing in overlapped layers along these bulkheads 7a.

If the target fluid in the parallelepiped-type treatment container is water, the fluid outlet can be installed at a height above the highest water level in the treatment container, and a position of the inlet can be set to be higher than that of the outlet, thereby eliminating a need for power to move the fluid within the treatment container.

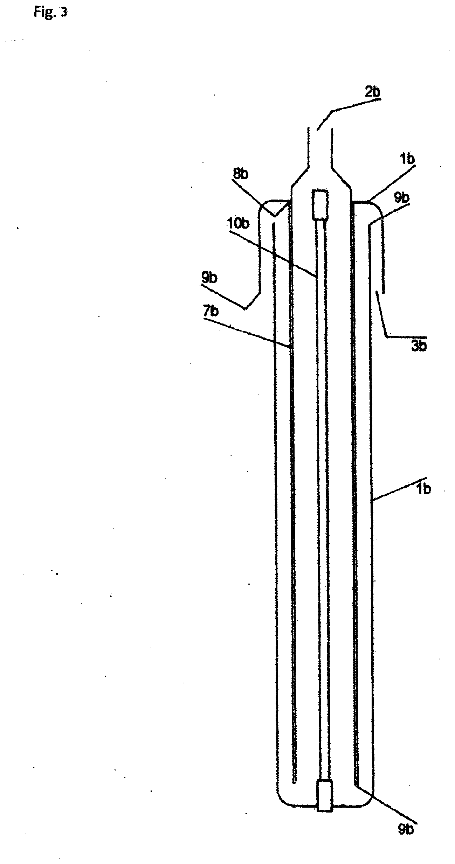

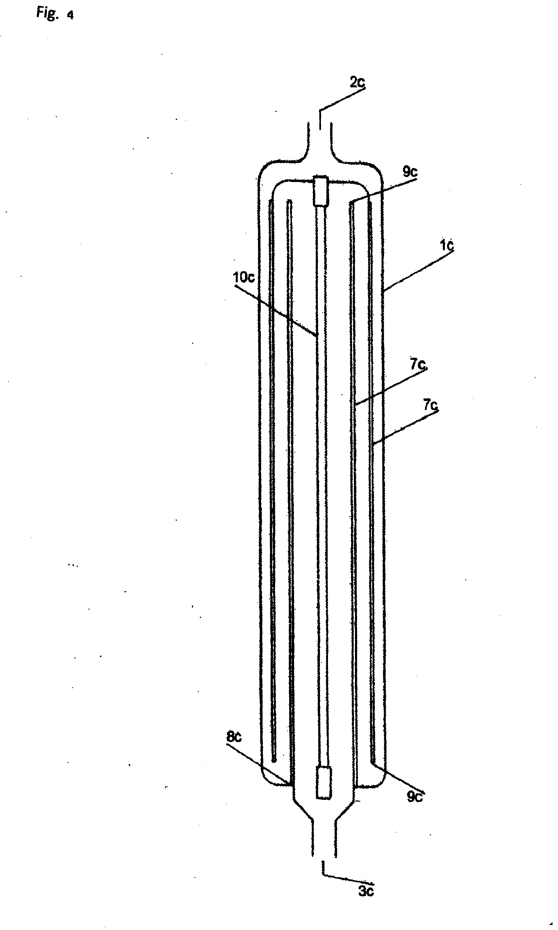

In order to obtain the fluid flow described in [0013] in the cylindrical treatment container, in the cylindrical treatment container having a cylindrical UVC lamp on the central axis of the inner wall of the main cylindrical part of the enclosure, as shown in Figs. 3, and 4, between the UVC lamp and the inner wall of the main cylindrical part of the treatment container, there is a cylindrical single bulkhead composed of a UVC highly transmissive material or a bulkhead having a plurality of different predetermined diameters on a concentric circle about the central axis of the main cylindrical portion of the treatment container; one end face of the bulkhead adheres to one end face inside the treatment container; the other end face of the bulkhead has a predetermined spaced opening through which fluid passes to the other end face inside the treatment container, and when there is a plurality of bulkheads, it has openings located between the end faces at mutually opposite side and the end faces of the inside of the treatment container between adjacent bulkheads.

Fig. 3 is a schematic view of a cylindrical treatment container when the target fluid is air. This includes a cylindrical enclosure 1b, one UVC lamp 10b on the circular central axis of the enclosure 1b, one cylindrical bulkhead 7b concentric with the central axis, an air outlet 2b at a center of one end face of the enclosure 1b, and an air intake opening 3b.

Fig. 4 is a schematic view of a cylindrical treatment container when the target fluid is water. This includes one UVC ramp 10c on a circular central axis of a cylindrical enclosure 1c, two cylindrical bulkheads 7c on different concentric circles of a related central axis, a water outlet 2c at a center of one end face of the enclosure 1c and a water inlet 3c at a center of the other end face.

In these parallelepiped-type and cylindrical-type treatment containers, the amount of UVC energy per unit volume of fluid received at the fluid outlet is a total amount of UVC energy received per unit volume of fluid in each fluid layer.

Conversely, the intensity of UVC irradiated at each fluid layer from the light source array surface or the light source to each fluid layer is absorbed and attenuated from an inside of the treatment container and the fluid inlet/outlet to the structure leading to the outside, but a large ratio of the attenuation is compensated slowing a flow speed by increasing the thickness in each flow layer, and lengthening a time for the fluid in the layer to receive UVC irradiation, and increasing an amount of UVC energy received per unit volume of fluid. However, a difference in thickness between adjacent fluid layers should be kept within a range where flow passage and stagnant areas described in [0007] above do not occur.

In the fabrication of such treatment containers, if the UVC reflectance of the inner wall of the enclosure is inadequate, an aluminum sheet having a UVC reflective surface (hereinafter, "reflective sheet") of around 90 percent is attached to the inner wall of the enclosure with its reflective surface facing the inside of the enclosure.

In attaching such reflective sheets, a method is adopted that also minimizes the obstruction to UVC irradiation by the parts that attach the bulkheads, or the frame that holds the bulkhead material when thin bulkhead material is used.

As a method, in the present invention, in a pair of inner walls of the enclosure that does not form an opening with a bulkhead, a reflective surface of each part of the inner wall opposite the light source is inserted between the light source array surface and the nearby bulkhead, between mutual bulkheads and between the inner wall opposing the light source the nearby bulkhead, and when the bulkhead that does not have a holding frame is approximately 5 mm to 15 mm, pull out the inside of the enclosure up to a position that covers the holding frame when using a bulkhead that has a holding frame, or by mounting by standing it, form a groove to insert a side end of the bulkhead between mutually adjacent reflective sheets. However, dimensions to raise the reflective sheet at the inside of the enclosure should be equal between each of the above.

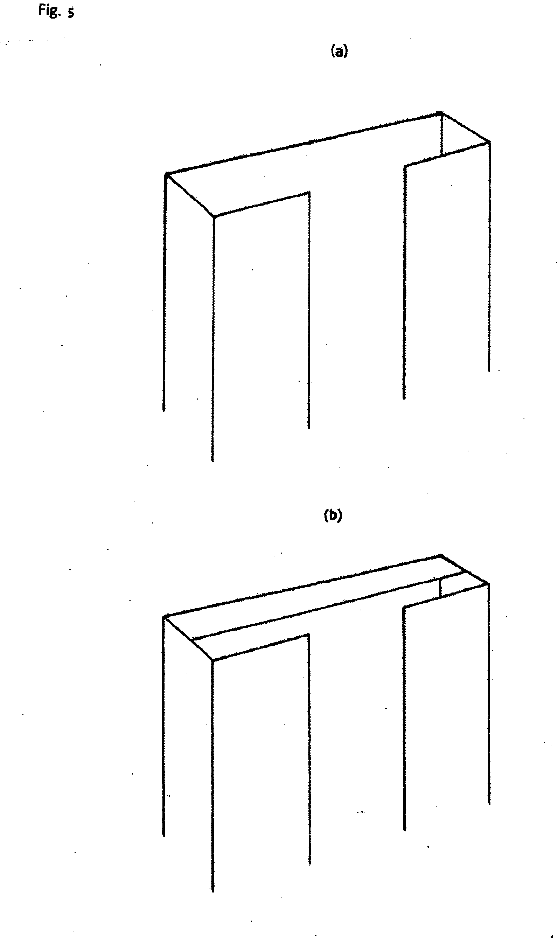

As one method of raising and attaching the reflective sheets to the inside of the enclosure in this manner, to the reflective sheet of each part of the inner wall described in [0025] above dispose an end 2 on a line of a predetermined width taken from a ridge line of a bent angle of an end 1 of a predetermined width that has been bent 90 degrees on the opposite side of the reflective surface of the reflective sheet, as shown in Fig. 5(a), and further 90 degrees inwardly, and bent 180 degrees including a bending angle from the reflective surface facing inward of the enclosure, and this end 2 is used as a screwing part to each part of the inner wall. A width of the edge 2 should be such that when each folded structure of the reflective sheet (hereinafter, "reflective body") formed in this way is screwed to each of the inner walls interposed with a spacer of a thickness of approx. 1 to 2 mm, a space of 5 mm or more will remain between the bended corner to the edge 2 and the spacer. Also, having a small width surface at the edge of the reflective body that is not bent, as shown in Figure 5(b), bent 90 degrees to an opposite side of the reflective surface of the reflective body, has an effect of increasing a rigidity of the reflective body.

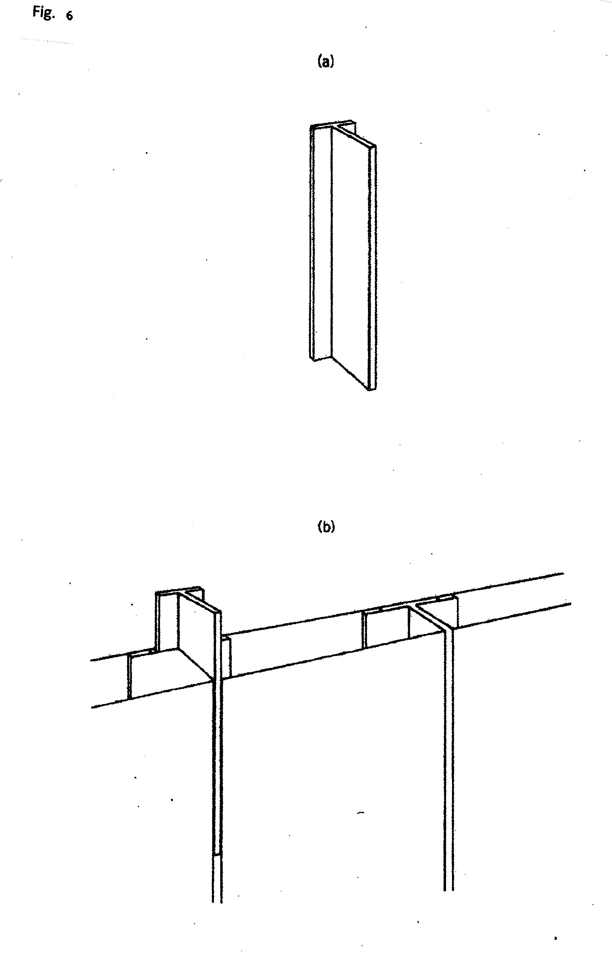

By forming a groove to insert the pair of ends at each bulkhead between adjacent reflective bodies, and any of a pair of T-shaped position stoppers with orthogonal cross-sections shown in Fig. 6(a) and inserting the position stoppers into the groove shown in Fig. 6(b) before or after the bulkhead, the reflective body formed as described in [0028] above and attached to the inner wall can fix a position of the bulkhead.

Both parallelepiped-type and cylindrical-type treatment containers are formed by being sandwiched at a 90-degree angle between the opening side of the bulkhead and the inner wall of the treatment container that touches an end surface of the opposite side, and two surfaces of the bulkhead. The inner corners of these that extend orthogonally to the fluid flow, are indicated by the symbols 8a, 8b, and 8c respectively in Figs. 1, 3 and 4, and they are labeled "orthogonal inside corners." Also, in parallelepiped-type treatment container and cylindrical-type treatment container in which the target fluid is air, the ends of the enclosure and the ends on the opening side of the bulkheads that are part of the fluid inlet constitution indicated by the symbols 9a and 9c in Figs. 1 and 3 are projections that extend orthogonally to the fluid flow and are called "outside corners." In cylindrical-type treatment containers in which the target fluid is water, only the end of the opening side of the bulkhead wall is an outside corner, indicated by the symbol 9c in Fig. 4.

These orthogonal inside corners and outside corners can cause stagnation or a delay at a small portion of the fluid flow in the vicinity thereof.

When attaching the reflective sheet described at [0025] between the light source array surface, the bulkhead, the orthogonal inside corner formed in the orthogonal angle formed in the orthogonal angle of the parallelepiped-type treatment container of [0030] and the inner wall of the processing container forming an opening with the septum or opposite to the inner wall and the light source surface, orthogonal angle formed between the light source and its immediate orthogonal angle, between the orthogonal angle, or between the inner wall sections having an orthogonal angle at one end, along the angle line of the interior of the housing that is the reflective angle side of the reflective angle side of the reflective sheet, the curved surface or plane is provided at the end of the orthogonal angle of the interior angle of the reflective angle of the reflective sheet of each section, and the sheet is attached to cover the orthogonal angle.

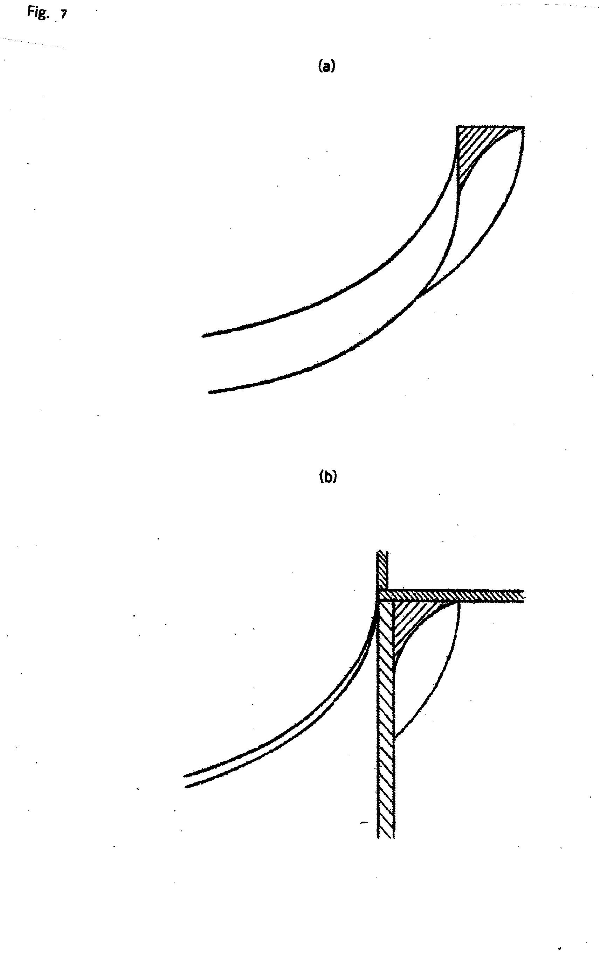

In addition to the measures described in [0032] above, another option is to cover the orthogonal inside corners of a parallelepiped-type treatment container with a long-axis body having various cross-sectional shapes that mitigate a sudden change in direction of the fluid caused by the orthogonal inside corners, or to cover the orthogonal inside corners of a cylindrical treatment container as shown in Fig. 7(b) with an annular body similar to an annular body depicted in Fig. 7(a).

For orthogonal outside corners, one option is to insert a tip of the orthogonal outside corner into a long-axis body or annular body whose cross-sectional shape perpendicular to an extending direction of the outside corners is a circle or similar thereto at the orthogonal outside corner, in order to mitigate an abrupt change of direction of the fluid at the outside corners and to generate a small flow on a vortex or around corners toward the bulkhead.

To prevent or reduce the stagnation or delay of flow at the orthogonal inside and outside corners, in addition to the measures described in [0032], [0033], and [0034] above, by disposing a structure of various cross-sectional shapes in front of the fluid flow at the orthogonal inside and outside corners, or by disposing many holes over a suitable range including the openings in the bulkhead that include a surface area of those holes, instead of the openings formed between the bulkheads and the inner wall of the treatment container, a turbulent and branch flow can be induced which is thought to involve the fluid near the outside corners or the fluid of the inside corners in the turbulent flow or branch flow.

When a bulkhead is included in the configuration of an orthogonal entry corner, or when an orthogonal exit corner is formed by a bulkhead, the structural materials described in [0033], [0034], and [0035] above should be made of materials with as high a UVC transmittance as possible, or materials with as high a UVC reflectance as possible.

### [Advantageous Effects of Invention]

A functional structure required in [0012] and [0013] above is attained by a basic structure of the UVC irradiation treatment container described in [0014] to [0036], the fabrication method of the treatment container based on this, and the functional reinforcement method of the treatment container. This provides a functional structure for continuous UVC irradiation treatment of fluids with high energy utilization efficiency, and this paves the way for development of various sterilizing devices and sterilization systems using UVC irradiation.

This divides the differences in UVC irradiation intensity into layers depending on the distance from the light source, reduces the differences in UVC irradiation intensity within each layer, and promotes uniformity in the UVC irradiation intensity received by bacteria and other microbes and viruses in the fluid. Since the amount of energy received is a product of irradiation time and intensity which is determined by a flow velocity of the fluid in each layer, the flow velocity of the fluid in each layer is determined by a predetermined treatment speed and a thickness or volume of each layer, and with this, the product of the time that a predetermined amount of fluid passes through the layer and the irradiation intensity within that layer is the amount of irradiation energy received by the fluid of a predetermined treatment volume in the layer. A total amount of UV energy received by a predetermined amount of fluid in a predetermined treatment volume is the sum of the amount of UV energy received by the same amount of fluid in each layer because of the functional structure of the invention.

This means that by setting a thickness of each layer in the treatment container, the amount of UV energy received by a predetermined amount of fluid from a light source within a predetermined time can be substantially increased compared to the same amount of energy received by the same amount of fluid from the same light source at the same time using a conventional single-layer treatment container.

### [Brief Description of Drawings]

Fig. 1 is an overhead view of a parallelepiped treatment container;
Fig. 2 is a conceptual view of air flow in a parallelepiped treatment container;
Fig. 3 is a schematic view of a cylindrical treatment container for air;
Fig. 4 is a schematic view of a cylindrical treatment system for water;
Fig. 5(a) is an overhead view of reflective body; 5(b) is an overhead view of reflective body with an additional bend;
Fig. 6(a) is an overhead view of T-shaped clasp; 6(b) is a view of insertion of T-shaped fastener; and
Fig. 7(a) is an overhead view of corner cover with annular body and bulkhead fastening; 7(b) is an overhead view of annular body installation.

### [Description of Embodiments]

When the target fluid in the cylindrical-type treatment container and parallelepiped-type treatment container of this invention is air, each container becomes a UVC irradiation air sterilizer by attaching an axial flow fan to the fluid outlet of the former and a cross fan to the fluid outlet of the latter.

The parallelepiped-type treatment container can also be used as a sterilizing air conditioner by connecting its air outlet to the air intake of an air conditioner with a duct.

When the target fluid of these containers is water, both cylindrical and parallelepiped containers are used in water treatment systems as sterilization units for water being fed by transport pipe.

In such a case, the fluid inlet and outlet of the cylindrical treatment containers are each connected to pipes, and the fluid inlet and outlet of the parallelepiped treatment container are set at the positions described in [0020] above, and water is poured into the inlets.

### [Example 1]

In this embodiment, the target fluid is air. In a stainless steel container with internal dimensions of 850 mm x 950 mm x 440 mm, the inner wall with dimensions of 850 mm x 950 mm is the light source mounting surface, the 850 mm x 440 mm inner wall is the top surface of the container and disposed at the end of the light source mounting surface side of the inner wall, is the air flow outlet described in [0015] and [0016]. The air inlet and outlet as described in [0015] and [0016] is disposed at the end of the inner wall opposing the light source of same inner wall.

For the UVC light source, 10 Philips UVC lamps, G30 T8 Bulb 30 Watt UVC Tube UV Output: 253.7 nm, are used. A reflective body for the UVC light source of the same length as the lamp, with a cross-section perpendicular to its long axis direction that is a phase including one focus of an ellipse; 10 emitters with a width of 80 mm outside an opening of the reflective body and an external dimension of 55 mm in a depth direction of the reflective surface, are connected side by side, and the external dimensions of the 850 mm x 950 mm parallelepiped unit are integrally formed into a reflective body with an external thickness of 55 mm. The reflective body is attached to the light source mounting surface, leaving a width of 25 mm from each side of the reflective body, and 10 UVC lamps are attached to this.

Three sheets of 50 µm thick film of AGC's product "EFCLEAN" (trade name), made of an ETFE material, attached to a stainless steel frame with an edge width of 25 mm and external dimensions of 950 mm x 850 mm, are used as bulkheads. The first bulkhead is mounted 80 mm from the light source array surface, the second bulkhead is 90 mm from the first bulkhead, the third bulkhead is 100 mm from the second bulkhead, and the third bulkhead is 115 mm from the inner wall opposing the light-source unit.

For the attachment of the reflective sheet to the inner wall of the container, at [0026], [0027], and [0028], a reflective body with a height raised 25 mm of the reflective surface is embedded between a first and a third bulkhead near the light-source array surface at the inner wall that has the air outlet, and at the inner wall opposite the inner wall, the reflective sheet with a 25 mm gap is inserted between the inner wall and the end of the reflective body of the lamp, filling the gap between the light source mounting surface and the end of the second bulkhead, so by attaching the same reflective body between the end of the second bulkhead and the inner wall opposing the light-source unit, a groove is formed into which the end of the second bulkhead is inserted.

The reflective body to be attached to a pair of inner walls that do not have an opening between them and the bulkheads has a 25 mm standing height by inserting a T-shaped bulkhead stopper as described at [0029], which is inserted into the 25 mm gap between the inner wall and the end of the lamp reflective body and is positioned to fill a space between the first bulkhead frame and the first bulkhead frame, and each gap between the first bulkhead frame and the second bulkhead frame, the second bulkhead frame and the third bulkhead frame, the third bulkhead frame and the inner wall opposing the light source is embedded, and a groove is formed to insert these bulkhead frames and the above T-shaped fasteners. The bulkhead is inserted following the T-shaped bulkhead positioning fasteners, or the fasteners are inserted following the bulkheads and the bulkheads are attached.

The air inlet is connected to a duct having the same rectilinear cross-sectional shape and dimensions as the inlet, which runs along an outer wall of the container to the outside of the inner wall opposing the light-source unit. At a position 30 cm below along the outer wall, a fan is attached cutting the duct 45 degrees toward the outside of the container, and a cross fan is attached to the air outlet to draw air and blow air continuously into the container, giving a function to blow air out and implement the air sterilizer.

### [Example 2]

The body of the treatment container in the embodiment has a structure that affixes the reflective sheet described at [0025] to the inner periphery of a cylindrical stainless steel container having an inner diameter of 120 mm and a height of 890 mm and a flat, bottom surface, and a lid having a cylindrical unit with an inner diameter of 80 mm and a height of 50 mm continuing on the cylindrical unit with an inner diameter of 150 mm and a height of 100 mm, is on the same axis as the cylinder and mounted apart from the mouth of the container by 14 mm. The internal structure is composed of a single lamp of the same specifications as the UVC lamp used at [0045] on the same central axis, and a quartz pipe bulkhead of 80 mm inner diameter, 86 mm outer diameter, and 890 mm height concentrically between the lamp and the inner container wall, with a 14 mm gap from the container bottom.

To install the UVC lamp, a socket cover for the lamp fastened by inserting into the center of the bottom of the container, and a plate arm that extends each 120 degrees from the socket cover to the center unit of the cylindrical unit with an inner diameter of 80 mm of the like is screwed from the outside of the cylindrical unit, and a lead wire is pulled to the outside along with the arm.

The bulkheads are attached by inserting the bulkheads into the three notches by screwing plate-shaped spacer having a perpendicular notch in contact with both the end surface and the peripheral surface in the same part across the lower end of the bulkhead and the peripheral surface and contacting with the bottom of the container and peripheral surface at a distance of 14 mm from the bottom surface and 17 mm from the peripheral inner surface of the container, into the inner wall of the container every 120 degrees. The upper part of the bulkhead is screwed so that the inner circumference of the annular body is positioned where the outer circumference of the end face of the bulkhead is in contact with the flat part where the inside diameter of the lid moves from 150 mm to 80 mm and the bulkhead fills the corner formed by the bulkhead and the annular body corner cover and bulkhead clamp shown in Figure 7(a), and the end of the bulkhead is inserted therein as shown in Fig. 7(b).

An axial fan is attached to the upper end of the cylindrical part with an inner diameter of 80 mm functioning to draw air out of the container. The opening between the container and the lid serves as an air inlet, and the entire unit is mounted on a vertical support stand to form a UVC irradiation air sterilization system.

### [Industrial Applicability]

Because of high anticipated contributions of this invention, the invention has a high possibility of being used in operating rooms, critical care units, nursing homes, and classrooms and the like.

### Reference Signs List

1a parallelepiped-type treatment container body
2a parallelepiped-type treatment container air outlet
3a air inlet for parallelepiped-type treatment container
4a air inlet for parallelepiped-type treatment container
5 light source mounting surface
6 light source array surface
7a parallelepiped-type treatment container bulkhead
8a orthogonal inside corner in parallelepiped-type treatment container
9a outside corner in parallelepiped-type treatment container
1b body for cylindrical-type treatment container targeting air
2b air outlet for cylindrical-type treatment container targeting air
3b air inlet for cylindrical-type treatment container targeting air
7b cylindrical-type treatment container bulkhead targeting air
8b inside corner of cylindrical-type treatment container targeting air
9b outside corner of cylindrical-type treatment container targeting air
10b UVC lamp for cylindrical-type treatment container targeting air
1c body for cylindrical-type treatment container targeting water
2c water flow outlet for cylindrical-type treatment container targeting water
3c water inlet for cylindrical-type-treatment container targeting water
7c cylindrical-type treatment container bulkhead targeting water
8c inside corner for cylindrical-type treatment container targeting water
9c outside corner for cylindrical-type treatment container targeting water
10c UVC lamp

## Claims

1. A UVC irradiation treatment container **characterized by** have a functional structure of a C region ultraviolet ray (hereinafter, "UVC") lamp or UVC LED lamp or UVC led chip attached toward an inner wall (hereinafter, "light source mounting surface") opposing a central direction of the UVC irradiation toward the light source mounting surface, substantially completely across one inner wall of the container that is optically blocked from the outside, a plurality or single bulkhead having a rectangular shape is parallel to the light source mounting surface, between the UVC irradiation side array surface of the light source (hereinafter "light source array surface") and the inner wall opposing the light source, and forms a predetermined space between the light source array surface and each of the inner walls opposing the light source, and between mutual bulkheads, one end surface of each bulkhead has an opening of a predetermined width therebetween the inner wall, and mutually opposing end surface sides between adjacent bulkheads, at the inner wall that opposes the inner wall that forms the opening between the light source array surface and the nearby bulkhead, and at the inner wall that opposes the inner wall that has an opening between the bulkhead and the light source mounting surface, and between the nearby bulkhead at the inner wall opposing the light source, or alternatively to that, has an opening that passes to an outside of the container at the opening of the nearby bulkhead at the inner wall opposing the light source and the end of the inner wall opposing the light source on the other side, one of these being a flow -out opening from the fluid container, and the other is a flow-in opening to the fluid container, for fluid that continuously passes through the inside of the container.

2. In a pair of inner walls that do not form an opening between them and a bulkhead, in claim 1, a UVC irradiation treatment container having a mechanism to insert a bulkhead between mutual reflective sheets (hereinafter, "reflective sheets") of each inner wall, by attaching at a predetermined standing height reflecting surfaces of aluminum sheets having a surface finished with highly UVC reflectance at each inner wall between a light-array surface and a nearby inner wall, between mutual bulkheads, and between an inner wall opposing the light-source unit and a nearby bulkhead.

3. A method for obtaining a rise of a reflective sheet according to claim 2, comprising a bent end 2 having a 180 degrees bend angle from the reflective surface toward an inner surface of the container, at an end 1 of a predetermined width bend at 90 degrees at an opposite side of a reflective surface of the reflective sheet, and a line having a predetermined width from a ridge line, on a reflective sheet of each inner wall according to claim 2, attaching the bent end 2 as a part to the inner wall disclosed in claim 2 of the reflective sheet directly or via a spacer.

4. The method for attaching a reflective sheet by disposing a bend surface folded to an inner side of the container to become the reflective surface side of the reflective sheet, wherein when attaching the reflective sheet according to claim 2 at each part of the inner wall sandwiched by the inside corners formed by the pair of inner walls forming an opening of a bulkhead according to claim 1 and claim 2, the light source array surface, a bulkhead, and each of the inner walls opposite the light source, along an end of the inside corner of each reflective sheet and along the inside corner line.

5. A UVC irradiation treatment container **characterized by** comprising a cylindrical UVC lamp at a center of a cylinder of a container having a cylindrical inner wall optically shielded from the outside; having one or a plurality of cylindrical bulkheads composed of a highly UVC permeable material between the lamp and the inner wall of the container, one end of the bulkhead having an opening between it and an end inner wall of the container and the other end in close contact with an end inner wall of the opposite side of the container; when there is a plurality of bulkheads, a position of the opening between the end inner wall of the container is reversed alternately between the bulkheads, and comprises a functional structure described above to implement UVC irradiation on a fluid passing through the container.
